(19) Europäisches Patentamt
European Patent Office
Office européen des brevets

(11) Numéro de publication: **0 259 227 B1**

(12) **FASCICULE DE BREVET EUROPEEN**

(45) Date de publication de fascicule du brevet: **27.12.91**

(51) Int. Cl.5: **C07D 211/34**, C07D 211/70

(21) Numéro de dépôt: **87401959.9**

(22) Date de dépôt: **02.09.87**

# (54) Dérivés de la benzhydryloxyéthyl-pipéridine, procédé d'obtention et compositions pharmaceutiques les contenant.

(30) Priorité: **05.09.86 FR 8612508**

(43) Date de publication de la demande: **09.03.88 Bulletin 88/10**

(45) Mention de la délivrance du brevet: **27.12.91 Bulletin 91/52**

(84) Etats contractants désignés: **AT BE CH DE ES FR GB GR IT LI LU NL SE**

(56) Documents cités:
**EP-A- 0 048 705**
**EP-A- 0 115 700**
**FR-A- 2 276 824**
**FR-A- 2 350 100**

(73) Titulaire: **Société anonyme: LES LABORATOIRES MERAM**
**4 Bld Malesherbes**
**F-75008 Paris(FR)**

(72) Inventeur: **Buzas, André**
**25, Route de Versailles**
**F-91570 Bièvres(FR)**
Inventeur: **Merour, Jean-Yves**
**216, Allée des Pervenches**
**F-45160 Olivet(FR)**
Inventeur: **Ollivier, Roland**
**94, Rue de Fauvettes**
**F-45160 Olivet(FR)**

(74) Mandataire: **Gillard, Marie-Louise et al**
**Cabinet Beau de Loménie 55, Rue d'Amsterdam**
**F-75008 Paris(FR)**

**EP 0 259 227 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention a pour objet des dérivés de la benzhydryloxyéthyl-pipéridine et leurs sels pharmaceutiquement acceptables. Elle concerne également les procédés pour l'obtention de ces dérivés ainsi que les compositions pharmaceutiques les contenant.

On connaît déjà des dérivés de la pipérazine 1,4-disubstituée, à savoir la propionyl-1(2-benzhydryloxyéthyl)-4 pipérazine et ses sels d'addition d'acides organiques ou inorganiques qui sont décrits dans le brevet FR 2.276.824 comme agents antitussifs.

D'autres dérivés de la pipérazine sont décrits dans le brevet FR 2.350.100 comme étant des agents anti-émétiques et spasmolytiques. Ces dérivés sont des pipérazines disubstituées en 1,4, dans lesquelles le substituant en position 1 est un groupe benzhydryloxy alkyle ou benzhydrylalkyle et le substituant en position 4 est un groupe 4-fluorobenzoylalkyle.

Enfin, le brevet EP 48.705 décrit des acides 2-(4-diphénylméthylène)-1-pipéridinyl)-acétiques et leur amides ayant des propriétés anti-histaminiques et spasmolytiques.

On a maintenant trouvé des nouveaux dérivés de la pipéridine qui présentent des propriétés pharmacologiques intéressantes et notamment des propriétés antihistaminiques et antispasmodiques.

Les composés selon l'invention répondent à l'une des formules ci-après:

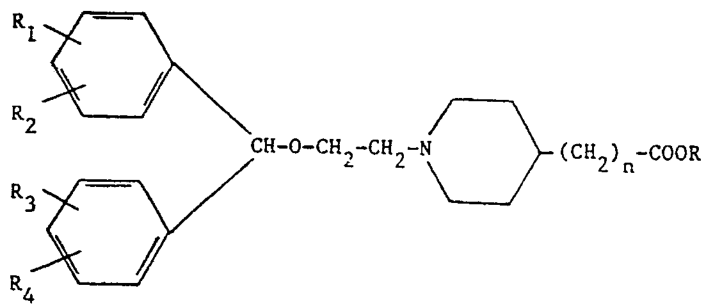

(I)

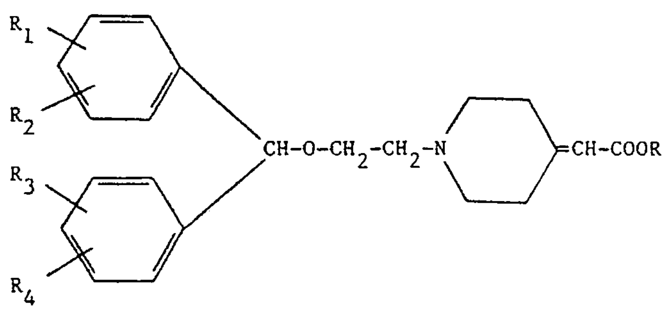

(II)

dans lesquelles:

- $R_1$, $R_2$, $R_3$ et $R_4$, identiques ou différents, représentent un atome d'hydrogène, un atome d'halogène, un groupe alkyle, un groupe alcoxy ou le groupe trifluorométhyle ;
- R représente un atome d'hydrogène, un cation, un groupe alkyle,
- n est égal à 0 ou 1.

Les composés particulièrement préférés aux fins de l'invention sont les composés ci-après :

- le 1-(benzhydryloxyéthyl) pipéridino-4-acétate d'éthyle.
- le 1-(benzhydryloxyéthyl)pipérid-4-ylidène acétate d'éthyle
- la 4-carbéthoxy-1-benzhydryloxy-éthyl-pipéridine et leurs sels pharmaceutiquement acceptables.

Les groupes alkyle selon l'invention sont les restes d'hydrocarbures aliphatiques contenant de I à 6 atomes de carbone, le groupe méthyle étant particulièrement préféré.

La présente invention concerne également les procédés pour l'obtention des composés de formules I et II qui sont décrits ci-après :

Procédé A : Obtention des composés de formule (I)

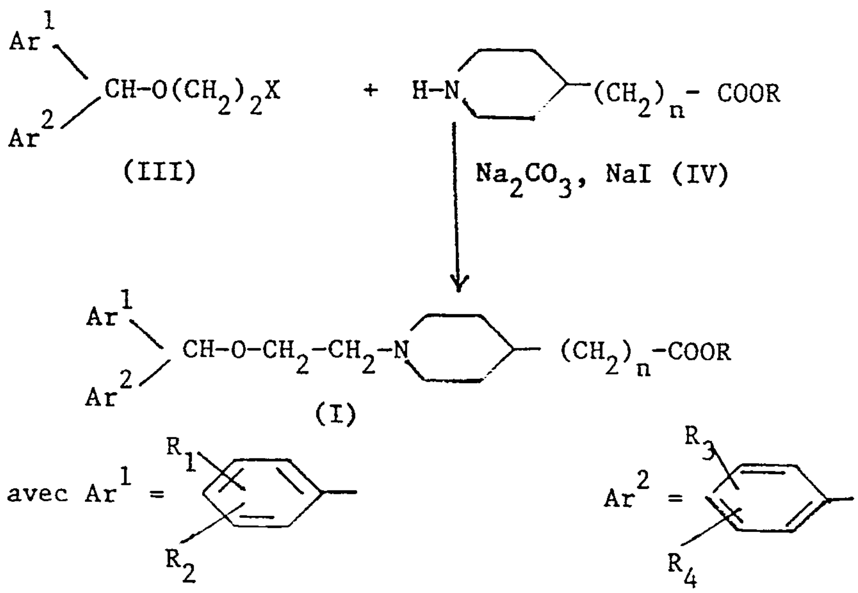


(III) (IV) (I)

avec

R, $R_1$, $R_2$, $R_3$, $R_4$ et n étant tels que définis précédemment.

Le procédé A, représenté par le schéma réactionnel ci-dessus, consiste à chauffer à reflux un dérivé halogéné de formule(III)dans laquelle $Ar^1$ et $Ar^2$ sont tels que définis ci-dessus et X est un atome d'halogène par exemple le chlore avec une amine de formule(IV)dans laquelle n = 0 ou 1, et R est tel que défini précédemment, dans un solvant aromatique, tel que le benzène ou le toluène ou dans la méthyléthylcétone et en présence d'un agent accepteur de l'acide halohydrique formé. On utilise avantageusement du carbonate de sodium ou de potassium. Une faible quantité de NaI peut être rajoutée pour accélérer la réaction.

Les composés de formule générale (IV), dans laquelle n = 1, peuvent être aisément obtenus par hydrogénation catalytique, en présence de Pd/C à température et pression ambiantes du dérivé benzylé (V) en solution dans un alcool primaire, tel que par exemple le méthanol ou l'éthanol.

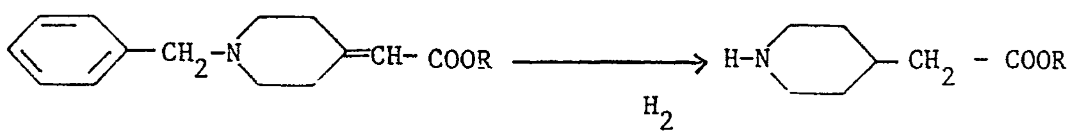


(V) (IV)

Les dérivés de formule générale (V) sont obtenus en faisant réagir le carbanion sodé du diéthylphosphonoacétate d'alkyle de formule (VI) sur la benzylpipéridone de formule (VII) dans le tétrahydrofuranne (THF) à 0 °C selon le schéma réactionnel suivant :

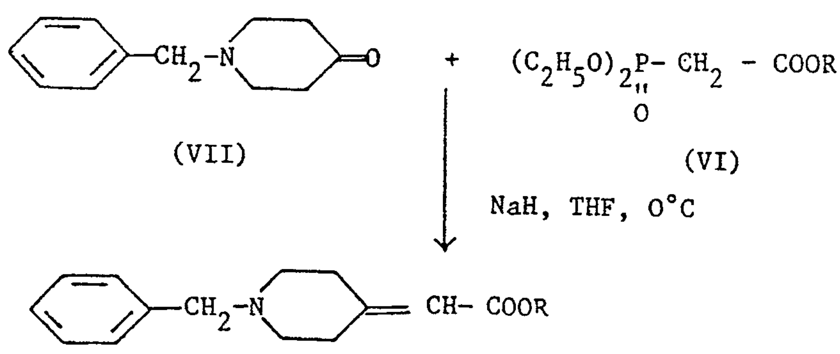


(VII) (VI)

(V)

Procédé B : obtention des composés de formule (I) dans laquelle n = 1.

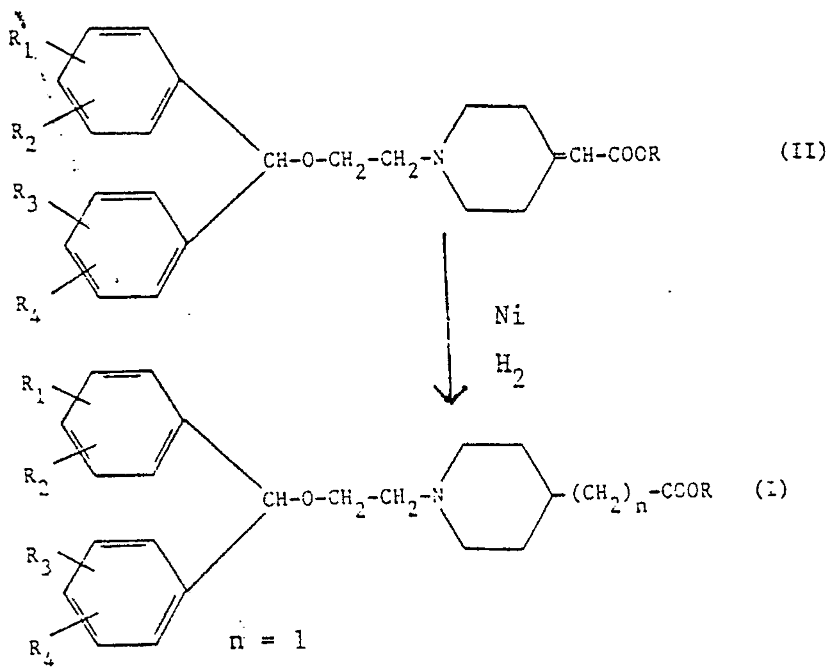


(II)

(I)

n = 1

Selon une variante de l'invention, les composés de formule(I) dans laquelle n = 1 peuvent être également préparés par hydrogénation catalytique, en présence de nickel de Raney, à température ambiante et sous pression d'hydrogène, du composé éthylénique correspondant de formule (II) dans un alcool primaire, tel que par exemple l'éthanol ou le méthanol.

Procédé C : obtention des composés de formule II

Ces composés peuvent être obtenus en faisant réagir le carbanion sodé du diéthylphosphonacétate d'alkyle (IX) sur la benzhydryloxyéthylpipéridone (VIII) dans le tétrahydrofuranne (THF) à 0 ° C.

Le schéma général de la réaction est le suivant :

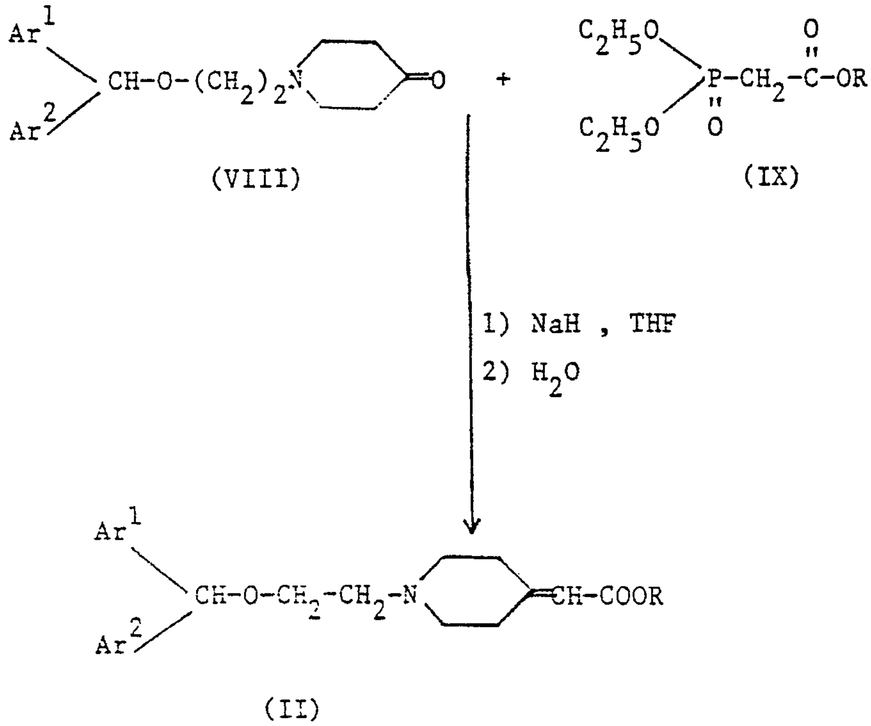


(VIII) (IX)

(II)

$Ar^1$, $Ar^2$ et, R étant tels que définis précédemment.

Les composés de formule (VIII) sont aisément préparés en chauffant à 80-90 °C les dérivés halogénés ou tosylés de formule (X) dans laquelle $Ar^1$ et $Ar^2$ sont tels que définis précédemment, avec le chlorhydrate de pipéridone (XI), en présence de deux équivalents de carbonate de sodium dans un solvant tel le diméthylformamide (DMF), selon la méthode décrite par L.D. WISE et collaborateurs (J. Med. Chem. 1985, 28, 1811-7).

Une faible quantité de NaI peut être rajouté pour accélérer la réaction.

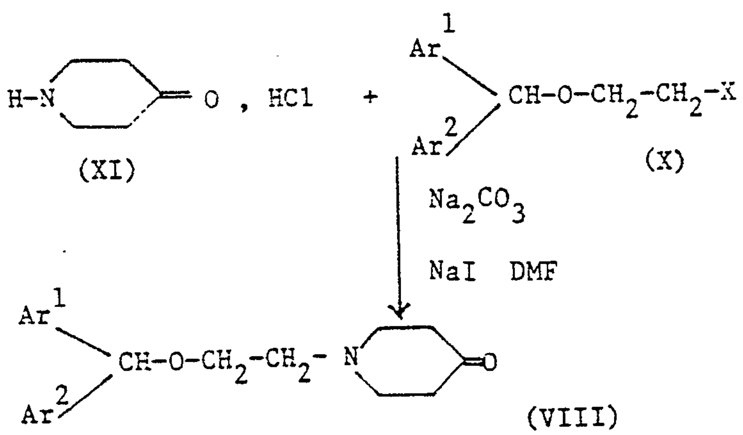


(XI) , HCl + (X) X = Cl, Br, OTs

(VIII)

Les sels d'addition d'acides des dérivés selon l'invention peuvent être obtenus par des procédés classiques avec des acides couramment utilisés pour obtenir des sels pharmaceutiquement acceptables, tels que par exemple l'acide acétique, l'acide chlorhydrique, l'acide bromhydrique, l'acide méthanesulfonique, l'acide maléique, l'acide tartrique ou l'acide fumarique.

Comme indiqué précédemment, les composés selon l'invention présentent des propriétés pharmacologiques intéressantes et notamment des propriétés antihistaminiques et antispamodiques et conviennent en particulier pour le traitement des états spasmodiques et des allergies. L'invention a donc également pour objet les compositions pharmaceutiques contenant à titre de principe actif un dérivé selon l'invention en combinaison avec un véhicule pharmaceutiquement acceptable.

Les compositions selon l'invention peuvent être des compositions administrables par voie orale ou

rectale ou par voie injectable. Elles peuvent se présenter sous forme de solutions, de comprimés, de pilules, de gélules, de suppositoires ou des compositions injectables.

L'invention va être maintenant décrite plus en détail par les exemples illustratifs 2 à 4 et 7 à 13, les exemples 1, 5 et 6 concernent l'obtention d'intermédiaires de synthèse qui ne font pas partie de l'invention, telle que revendiquée.

Les dérivés préparés ont été identifiés et caractérisés grâce à l'étude de leurs spectres RMN et infrarouge ainsi qu'à leur analyse élémentaire et sont désignés dans les essais pharmacologiques par les références internes de la demanderesse.

Exemple 1:Préparation de la (1-benzhydryloxyéthyl)pipéridine-4-one)
(formule VIII : $R_1 = R_2 = R_3 = R_4 = H$)

Dans un réacteur de 500 ml on a placé 10 g de 1-(benzydryloxy)-2-chloroéthane, 7,65 g de chlorhydrate de pipéridone, en solution dans 200 ml de diméthylformamide. On a ajouté 8,52 g de $Na_2CO_3$ et, 0,4 g de NaI. On a porté le mélange à une température de 80-90° C pendant 15 heures. Après refroidissement et filtration on a repris par 200 ml d'eau et on a extrait par 3x50 ml de toluène. La phase toluénique a été lavée par 2x50 ml d'acide chlorhydrique 0,1N. On a alcalinisé par du $NaHCO_3$ solide en présence de 50 ml d'eau.

Après décantation, on a séché et on a évaporé le solvant. On a obtenu ainsi 11,4 g de produit de formule générale $C_{20}H_{23}NO_2$.

Spectre de RMN (solvant $CDCl_3$, référence TMS) :

2,3 ppm (m), 4H, $CH_2$-C=O ; 2,8 ppm (m), 6H, $\underline{C}H_2$-N ; 3,5 ppm (t), 2H, $CH_2$-O ; 5,3 ppm (s), 1H, CH-O ; 7,1 ppm (m), 10H $\bar{\emptyset}$.

EXEMPLE 2 : Préparation de 1-(benzhydryloxyéthyl)pipérid-4-ylidène acétate d'éthyle selon le procédé C
(formule II : $R_1 = R_2 = R_3 = R_4 = H$ ; $R = C_2H_5$)

Dans un réacteur on a placé 0,6 g de NaH préalablement lavé par 2x5 ml de tétrahydrofuranne anhydre (THF). On a ajouté 50 ml de THF , puis on a additionné, goutte à goutte, à 20° C une solution de diéthylphosphonoacétate d'éthyle dans 30 ml de THF. On a agité pendant 10 min à cette température.

On a refroidi alors le mélange et on a ajouté lentement à 0° C la 1-(benzhydryloxyéthyl)pipéridin-4-one obtenue selon l'exemple 1 en solution dans 20 ml de THF. On a laissé le mélange réactionnel revenir à température ambiante et on a agité pendant 2 heures. Après évaporation du solvant, l'huile a été reprise par 100 ml d'eau. On a extrait par 3x50 ml de $CH_2Cl_2$, on a séché et on a évaporé le solvant . On a obtenu ainsi 8,2 g d'huile jaune. Formule générale $C_{24}H_{29}NO_3$.

Spectre de RMN (solvant $CDCl_3$, référence TMS) :

1,2 ppm (t), 3H, $OCH_2$-$CH_3$ ; 2,5 ppm (m), 10H, $CH_2$-N et $CH_2$-C= ; 3,4 ppm (t), 2H, CH-O-$\underline{CH_2}$ ; 4,0 ppm (q), 2H, C-$CH_2$ ; 5,1 ppm (s), 1H, CH-O ; 5,4 ppm (s), 1H, CH=C ; 7,0 ppm (m) , 10H, $\emptyset$.

Spectre infrarouge

1610 $cm^{-1}$ (C=C) ; 1660 $cm^{-1}$ (C=O-)

EXEMPLE 3 : Préparation du maléate du 1-(benzhydryloxyéthyl)-pipéridino-4-acétate d'éthyle selon le procédé B
(formule I : $R_1 = R_2 = R_3 = R_4 = H$ $R = C_2H_5$ et $n = 1$)

Dans un autoclave on a placé 3,52 g de nickel de Raney en suspension dans 80 ml d'éthanol anhydre. On a ajouté 7,6 g de 1-(benzhydryloxyéthyl)pipérid-4-ylidène acétate d'éthyle (II) obtenu selon l'exemple 2. On a hydrogéné sous 100 kg de pression à température ambiante pendant 24 heures. On a filtré le catalyseur et on a évaporé le solvant . On a obtenu ainsi 7,5 g de produit.

On a préparé le maléate en faisant réagir l'huile obtenue, dissoute dans l'éther, avec 1,5 g d'acide maléique en solution dans 2 ml d'éthanol absolu. En fin d'addition on a refroidi et on a essoré. On a obtenu 9 g de solide de point de fusion F = 76° C et de formule brute $C_{24}H_{31}NO_3$, $C_4H_4O_4$.

Spectre de RMN (base en solution dans $CDCl_3$, référence TMS) :

1,2 ppm (t), 3H, $CH_3$-C ; 1,3 à 2,0 ppm (m), 5H, $CH_2$-C,. CH-C ; 2,2 ppm (t), 4H, $CH_2N$ ; 2,6 ppm (t), 2H, $OCH_2$-$CH_2$-N, ; 2,8 ppm (d), 2H, $CH_2$-CO ; 3,5 ppm (t), 2H, $OCH_2$ ; 4,0 ppm (q), 2H, $COOCH_2$ ; 5,2 ppm (s), 1H, CHY O ; 7,0 ppm (m), 10H, Ø.

Spectre IR du sel (1% dans le KBr)

1730 $cm^{-1}$ (C=O), 1740 $cm^{-1}$ (C=O), 1590 $cm^{-1}$ (C=C), 2500 $cm^{-1}$ (N±H).

EXEMPLE 4 : Préparation du fumarate de la 4-carbéthoxy-1-benzhydryloxyéthyl-pipéridine selon le procédé A
(formule I : $R_1$ = $R_2$ = $R_3$ = $R_4$ = H ; R = $C_2H_5$ ; n = o)

Dans un réacteur, on a placé 14 g de 1-benzhydryloxy-2-chloro-éthane, 8 g d'isonipécotate d'éthyle en solution dans 140 ml de méthyléthylcétone. On a ajouté 11,8 g de carbonate de potassium et 0,2 g de KI.

On a chauffé à reflux pendant 16 heures.

Après refroidissement on a filtré et on a évaporé les solvants. On a repris l'huile visqueuse par 80 ml d'eau et, on a extrait par 3x40 ml de $CH_2Cl_2$. On a filtré et on a évaporé le solvant. On a obtenu 18,5 g de produit. On a préparé le fumarate en faisant réagir le produit obtenu avec 5,8 g d'acide fumarique en solution dans 2 ml d'éthanol anhydre. On a essoré et on a séché à l'étuve. On a obtenu un solide de formule brute $C_{23}H_{29}NO_3$, $C_4H_4O_4$.

Spectre de RMN de la base (en solution dans $CDCl_3$, référence TMS ) :

1,2 ppm (t) 3H,

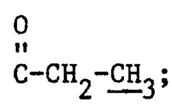

1,8 ppm (m), 4H, $CH_2$-C ; 2,1 ppm (m) , 1H, CH-COO ; 2,6 ppm (m), 6H, $CH_2N$ ; 3,4 ppm (t), 2H, $CH_2O$ ; 4,0 ppm (q), 2H, $CH_2OC$=O ; 5,2 ppm (s), 1H, CH-O ; 7,0 ppm (m), 10H, Ø .

EXEMPLE 5 : Préparation du 1-benzyl-pipérid-4-ylidène acétate d'éthyle
(formule V : R = $C_2H_5$ ; n = 1)

Dans un réacteur de 500 ml on a placé 7,92 g de NaH en suspension dans 150 ml de THF anhydre. On a additionné, goutte à goutte à 20° C, une solution de 73,9 g de diéthylphosphonoacétate d'éthyle dans 80 ml de THF. On a laissé sous agitation pendant 15 min, puis on a refroidi, à 0° C. On a ajouté alors, goutte à goutte en 20 min, une solution de benzylpipéridone (56,7 g) dans 80 ml de THF.
On a laissé sous agitation pendant une heure à température ambiante. (le mélange réactionnel est devenu gélatineux).

On a évaporé le solvant sous pression réduite et, on a repris par 150 ml d'eau. On a extrait par 3x80 ml de $CH_2Cl_2$. On a acidifié à pH 6 par HCl à 5%, on a décanté, séché, et évaporé le solvant . On a recueilli 61,5 g d'huile qui a été utilisée brute dans l'exemple suivant.

Spectre de RMN : (en solution dans $CDCl_3$, référence TMS)

1,2 ppm (t), 3H, COC-$CH_3$ ; 2,4 ppm (m), 8H, N-($CH_2$-$CH_2$) ; 3,4 ppm (s), 2H, Ø-$CH_2$, 4,0 ppm (q), 2H, O-$CH_2$-C ; 5,5 ppm (s), 1H, CH=C ; 7,1 ppm (m) , 5H, Ø .

EXEMPLE 6 : Préparation de pipéridino-4-acétate d'éthyle
(formule IV : R = $C_2H_5$ ; n = 1)

On a effectué l'hydrogénation du 1-benzylpipéridylidène acétate d'éthyle (21 g) dans une solution d'éthanol absolu (60 ml), en présence de 1,6 g de Pd/C à 10% et de 6,5 ml d'une solution éthanolique

d'acide chlorhydrique 13 N à température et pression ambiantes.

On a filtré le catalyseur, et on évaporé les solvants. Le solide a été dissous dans 40 ml d'eau. Après neutralisation par $NaHCO_3$ on a extrait au $CH_2Cl_2$ (4x50 ml). On a séché et on a évaporé le solvant . On a obtenu ainsi 9,6 g de produit de formule générale $C_9H_{17}NO_2$.

Spectre de RMN (solvant $CDCl_3$, référence TMS) :

1,2 ppm (t), 3H, $CH_3$ ; 1,3 à 3,2 ppm (m) 10H, NH,$NCH_2$-$CH_2$-CH ; 2,1 ppm (d), 2H,

$$CH_2\underset{\underset{O}{\|}}{C}\ ;$$

4,1 ppm (q), 2H, $OCH_2$

EXEMPLE 7 : Préparation du 1-(benzhydryloxyéthyl)-pipéridino-4-acétate d'éthyle (formule I : $R_1 = R_2 = R_3 = R_4 = H$ ; $R = C_2H_5$ ; $n = 1$)

En faisant réagir le 1-(benzhydryloxy)-2-chloro-éthane de formule (III) avec le pipéridino-4-acétate d'éthyle obtenu selon l'exemple 6 ci-dessus dans du toluène, en présence de carbonate de potassium et de NaI, on peut obtenir selon le procédé A le composé de l'exemple 3.

EXEMPLES 8 à 13 :

En opérant selon les modes opératoires décrits dans les exemples 2 et 3 ci-dessus on a obtenu les composés de formules I et II indiqués dans le tableau ci-après:

TABLEAU I

| Ex.No. | Dérivé de formule | $R_1$ | $R_2$ | $R_3$ | $R_4$ | n | R | T° C | exemple de préparation |
|---|---|---|---|---|---|---|---|---|---|
| 8 | I | H | 4-$CH_3$ | H | H | 1 | $C_2H_5$ | 85° (a) | 3 |
| 9 | I | H | 4-Cl | H | H | 1 | $C_2H_5$ | (a) | 3 |
| 10 | I | H | 4-$CH_3$ | H | 4-$CH_3$ | 1 | $C_2H_5$ | (a) | 3 |
| 11 | II | H | 4-Cl | H | H | - | $C_2H_5$ | (b) | 2 |
| 12 | II | H | 4-$CH_3$ | H | 4-$CH_3$ | - | $C_2H_5$ | (b) | 2 |
| 13 | II | H | 4-$CH_3$ | H | H | - | $C_2H_5$ | (a) | 2 |

(a) maléate
(b) fumarate

Les spectres RMN de ces composés sont donnés ci-après : (solvant $CDCl_3$; référence TMS)

Dérivé 8 1,2 ppm (t), 3H, $CH_3$-C ; 1,3 à 2,0 ppm (m), 5H, $CH_2$-C,CH-C ; 2,2 ppm (t), 4H, H-$CH_2$-C ; 2,3 ppm (s), 1H, $CH_3$Ø ; 2,6 ppm (t), 2H, C-$CH_2$-N ; 2,8 ppm (d), 2H,

$$CH_2\underset{\underset{O}{\|}}{C}-O$$

3,5 ppm (t), 2H, O-$CH_2$ ; 4,0 ppm (q), 2H,

$$\underset{\underset{O}{\|}}{C}-OCH_2\ ;$$

5,2 ppm (s), 1H, CH-O : 7,0 ppm (m), 9H, Ø.

Dérivé 9 1,2 ppm (t), 3H, $CH_3$C ; 1,3 à 2,0 ppm (m), 5H, $CH_2$-C,CH-C ; 2,2 ppm (t), 4H, $NCH_2$C ;

2,6 ppm (t), 2H, $CCH_2$-N ; 2,8 ppm (d), 2H,

$$CH_2\ \underset{\underset{O}{\parallel}}{C}-O\ ;$$

3,5 ppm (t), 2H, $OCH_2$ ; 4,0 ppm (q). 2H,

$$\underset{\underset{O}{\parallel}}{C}-OCH_2\ ;$$

5,2 ppm (s), 1H, CH-O ; 7,1 ppm m), 9H, Ø.

Dérivé <u>10</u> 1,2 ppm (t), 3H, $CH_3C$ ; 1,3 à 2,0 ppm, 5H, $CH_2$-C, CH-C ; 2,2 ppm (s), 6H, $CH_3$ Ø ; 2,2 ppm (t), 4H, N-$CH_2$-C ; 2,6 ppm (t), 2H, C-$CH_2$-N ; 2,8 ppm (d), 2H,

$$CH_2-\underset{\underset{O}{\parallel}}{C}-O\ ;$$

3,5 ppm (t) 2H, $OCH_2$ ; 4,0 ppm (q), 2H,

$$\underset{\underset{O}{\parallel}}{C}-OCH_2\ ;$$

5,2 ppm (s), 1H, CH-O ; 7,0 ppm (m) 8H, Ø.

Dérivé <u>11</u> 1,2 ppm (t), 3H, $CH_3$-C ; 2,0 à 3,0 ppm (m), 10H, N-$CH_2$-$CH_2$-C,$CH_2$ N ; 3,5 ppm (t), 2H, O-$CH_2$ ; 4,0 ppm (q), 2H, $COOCH_2$ ; 5,2 ppm (s), 1H, CH-O ; 5,5 ppm (s), 1H, C = CH ; 7,1 ppm (m), 9H, Ø.

Dérivé <u>12</u> 1,2 ppm (t), 3H, $CH_3C$ ; 2,1 à 3,1 ppm (m), 10H, C-$CH_2CH_2$-$NCH_2$ ; 2,2 ppm (s), 6H, $CH_3$Ø ; 3,5 ppm (t), 2H, $OCH_2$ ; 4,0 ppm (q), 2H,

$$\underset{\underset{O}{\parallel}}{C}-OCH_2\ ;$$

5,1 ppm (s), 1H, CHO ; 5,4 ppm (s), 1H,

$$C{=}CH-\underset{\underset{O}{\parallel}}{C}-O\ ;$$

7,0 ppm (m), 8H, Ø.

Dérivé <u>13</u> 1,2 ppm (t), 3H, $CH_3$-C ; 2,0 à 3,2 ppm, 10H, $CCH_2CH_2NCH_2$; 2,2 ppm (s), 3H, $CH_3$-Ø ; 3,5 ppm (t), 2H, $OCH_2$ ; 4,0 ppm (q), 2H,

$$\underset{\underset{O}{\parallel}}{C}-OCH_2\ ;$$

5,2 ppm (s), 1H, CH-O ; 5,5 ppm (s), 1H

$$C{=}CH-\underset{\underset{O}{\parallel}}{C}-O\ ;$$

7,1 ppm (m), 9H, Ø.

I -ESSAIS DE TOXICITE

La toxicité des composés de l'invention a été déterminée par les tests ci-après :

A. Détermination de la dose létale cinquante ($DL_{50}$) chez la souris.

Les dérivés étudiés ont été administrés par voie intrapéritonéale et per os à des groupes composés de cinq souris mâles et cinq souris femelles, à raison de 0,1 ml pour dix grammes de poids corporel.

Les doses suivantes ont été utilisées :

- pour la voie intrapéritonéale : 100-150-200 300 et 400 mg/kg
- pour la voie per os : 400 - 500 - 600 - 700 - 750 et 800 mg/kg.

La $D.L._{50}$,évaluée à partir de la mortalité observée, est indiquée dans le tableau II ci-après :

| | TABLEAU II | |
|---|---|---|
| DERIVE | $DL_{50}$ (I.P) mg/kg | $DL_{50}$ (P.O.) mg/kg |
| Exemple 3 (BM 113)<br>Exemple 2 (BM 138)<br>Exemple 4 (BM 159) | 160 mg/kg<br>300 mg/kg<br>456 mg/kg | 585 mg/kg |

B. Détermination de la dose minimale mortelle (D.M.M.) Chez le rat.

Les dérivés étudiés ont été administrés par voie intrapéritonéale chez le rat à deux groupes composés de 5 mâles.

Les doses suivantes ont été utilisés : 75 et 150 mg/kg.

La D.M.M. évaluée à partir de la mortalité observée, est indiquée dans le tableau III ci-après :

TABLEAU III

| DERIVE | DMM mg/kg I.P. |
|---|---|
| Exemple 3 (BM 113) | 75 mg/kg |

II - ESSAIS PHARMACOLOGIQUES

Les propriétés pharmacologiques des composés de l'invention ont été déterminées en utilisant les tests ci-après :

Protocoles d'essais

A- Etude de la motilité spontanée

L'activité motrice des souris a été déterminée à l'aide de l'actimètre photo-électrique de Boissier et Simon.

Les souris sont placées par cinq dans une boîte fermée par un couvercle, et traversée par deux rayons lumineux perpendiculaires que les souris coupent en se déplaçant.

Ces déplacements sont comptabilisés par un compteur relevé après trente minutes et une heure.

B- Comportement d'exploration

Trente minutes après l'administration intrapéritonéale des dérivés selon l'invention, chaque souris est placée sur une planche à trous automatisée pendant cinq minutes, et on note, minute par minute, le nombre de trous explorés.

Une dose efficace 50 peut être calculée en fonction des résultats obtenus.

C- Interaction sur l'hypermotilité provoquée par la dexamphétamine

On recherche un éventuel antagonisme de l'hyperactivité induite par la dexamphétamine administrée par voie intrapéritonéale à la dose de 3,5 mg/kg.

Les souris sont placées par cinq dans l'actimètre photoélectrique de Boissier et Simon composé de six boîtes fermées par un couvercle et traversées par deux rayons lumineux perpendiculaires que les souris coupent en se déplaçant.

Ces déplacements sont comptabilisées par un compteur qui est relevé après trente et soixante minutes.

D- Action myorelaxante(test de traction)

Ce test apprécie la présence ou l'absence de redressements d'une souris présentée par ses pattes antérieures à un fil métallique horizontal.

On note le nombre de souris qui ne peuvent accrocher au fil une de leurs pattes postérieures avant cinq secondes. Une dose efficace 50 peut être calculée en fonction des résultats obtenus.

E- Interaction avec le pentobarbital

On recherche une éventuelle augmentation du sommeil barbiturique en administrant par voie intrapéritonéale le produit à tester cinq minutes avant l'injection intrapéritonéale du pentobarbital (37,5 mg/kg).

Une dose efficace 50 peut être calculée en fonction des résultats obtenus.

F-Activité analgésique périphérique

Une douleur péritonéale de la souris est provoquée par injection intrapéritonéale de phénylbenzoquinone (PBQ). On recherche la diminution du syndrome douloureux caractérisé par une torsion abdominale à l'aide d'une injection du produit à essayer trente minutes avant l'administration de la P.B.Q.

La dose efficace 50 est calculée en fonction du pourcentage de diminution du syndrome douloureux par rapport aux témoins.

G- Activité analgésique centrale

On recherche une augmentation du temps de passage sur une plaque chauffée à 60° chez les souris traitées par le produit à essayer, trente minutes avant le début de l'essai.

La dose efficace cinquante est calculée en fonction du pourcentage d'augmentation du temps passé sur la plaque chauffante (léchage des pattes ou éventuels sauts).

H- Interaction avec l'oxotrémorine

L'oxotrémorine étant un agoniste des récepteurs cholinergiques, on peut penser que les substances qui antagonisent les tremblements, l'hypothermie et les signes périphériques (salivation, pilo-erection) induits par ce produit sont des anticholinergiques.

Le produit à essayer est administré trente minutes avant l'injection intrapéritonéale de l'oxotrémorine.

I- Etude sur organe isolé (Iléon de cobaye).

Recherche d'un antagonisme vis-à-vis des contractions induites par le chlorhydrate d'histamine sur l'iléon isolé de cobaye maintenu à 35° C dans le liquide de tyrode.

J- Bronchospasme par inhalation d'une solution d'histamine

Les cobayes sont placés dans une enceinte fermée où l'histamine est aérolisée, et seuls sont retenus ceux qui montrent des signes très nets d'asphyxie dans les quatre minutes.

La substance à étudier est administrée à des lots de cobayes trente minutes avant un nouveau passage dans l'enceinte pour contrôler la résistance à l'histamine. Un cobaye est considéré comme protégé s'il résiste pendant dix minutes à l'aérosol d'histamine sans montrer de signe d'asphyxie.

Une dose efficace 50 est calculée en fonction des résultats obtenus.

K- Activité antihistaminique.

- Recherche de la dose protectrice chez cinquante pour cent des cobayes vis-à-vis d'une dose mortelle d'histamine.
- Administration du produit trente minutes avant l'injection intraveineuse de chlorhydrate d'histamine (1 mg/kg). La dose efficace 50 est calculée en fonction des résultats obtenus.

L-Recherche de la dose protectrice chez 50% des cobayes vis-à-vis du choc anaphylactique.

Ce test a été réalisé selon le protocole ci-après :

- Administration aux cobayes par voie intrapéritonéale de 2ml de sérum de cheval à 20% dans le soluté physiologique pendant trois jours : J.1 - J.3 et J.5.
- Action déclenchante : à J.15, administration par voie orale du produit à tester, 45 minutes avant l'injection de 0,5 ml par voie intraveineuse de sérum de cheval pur.

On a obtenu avec le composé de l'exemple 3 (BM 113) une $DE_{50}$ de 0,829 mg/kg (P.O).

Résultats

Les résultats obtenus sont consignés dans le tableau IV ci-après. Ces résultats montrent que les composés de l'invention présentent une activité antihistamique et une activité antispasmodique.

Essais comparatifs :

A titre de comparaison, on a effectué les tests ci-dessus avec la Terfenadine, comme composé de l'art antérieur ; ce composé a donné les résultats ci-après dans les différents tests définis ci-dessus:

SOURIS

| | |
|---|---|
| Toxicité par voie intrapéritonéale | $D.L._{50}$ = 100 $mg.kg^{-1}$ I.P. |
| Activité motrice | Diminution importante à 25 $mg.kg^{-1}$ I.P. |
| Comportement d'exploration | $D.E._{50}$ = 37 $mg.kg^{-1}$ I.P. |
| Action myorelaxante - Test de traction | $D.E._{50}$ = 31 $mg.kg^{-1}$ I.P. |
| Interaction avec le pentobarbital | $D.E._{50}$ = 25 $mg.kg^{-1}$ I.P. |
| Activité analgésique périphérique | $D.E._{50}$ = 3,1 $mg.kg^{-1}$ I.P. |

COBAYE

| | |
|---|---|
| Bronchospasme par voie orale | $D.E._{50}$ = 1,60 $mg.kg^{-1}$ P.O. |
| Choc histaminique par voie orale | $D.E._{50}$ = 2,58 $mg.kg^{-1}$ P.O. |

TABLEAU IV

| Dérivé de l'invention | Activité motrice | Comportement d'exploration | Hypermotilité à la dexamphétamine | Action myorelaxante Test de traction | Interaction avec le pentobarbital | Activité analgésique périphérique | Activité analgésique centrale | Interaction avec l'oxotrémorine | Test "in vitro" | Bronchospame | | Choc histaminique |
|---|---|---|---|---|---|---|---|---|---|---|---|---|
| | (souris) mg/kg I.P. | (souris) mg/kg I.P. | (souris) mg/kg I.P. | (souris) mg/kg I.P. | (souris) mg/kg I.P. | (souris) $DE_{50}$ mg/kg I.P. | (souris) | (souris) | (iléon isolé) $DE_{50}$ µg | (cobaye) $DE_{50}$ mg/kg I.P. | P.O. | (cobaye) $DE_{50}$ mg/kg P.O. |
| Ex. 3 (BM 113) | aucune modification jusqu'à 6,25 | aucune modification à 25,0 | aucune modification à 12,5 | aucune modification à 25,0 | aucune augmentation du sommeil barbiturique | 1,9 | aucune activité | aucun antagonisme | 8,0 | 0,097 | 0,223 | 0,061 |
| Ex. 2 (BM 138) | diminution à 11 | aucune modification à 25,0 | aucune modification | aucune modification à 25,0 | augmentation du sommeil barbiturique | 1,9 | aucune activité | aucun antagonisme | > 10 | 0,65 | 0,21 | 0,25 |
| Ex. 4 (BM 159) | aucune modification | diminution à 25,0 | aucune modification | aucune modification | augmentation à 25,0 | faible analgésie | aucune activité | aucun antagonisme | 5 | 0,46 | 0,49 | 0,334 |

## Revendications

**Revendications pour les Etats contractants suivants : BE, CH, DE, FR, GB, IT, LI, LU, NL, SE**

**1.** Dérivés de benzhydryloxyéthyl-pipéridine caractérisés en ce qu'ils répondent à l'une des formules ci-

après :

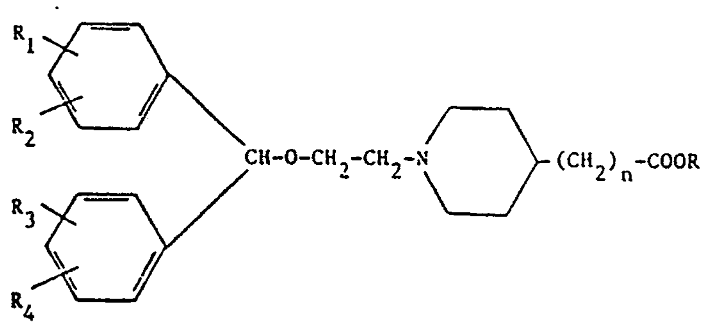

(I)

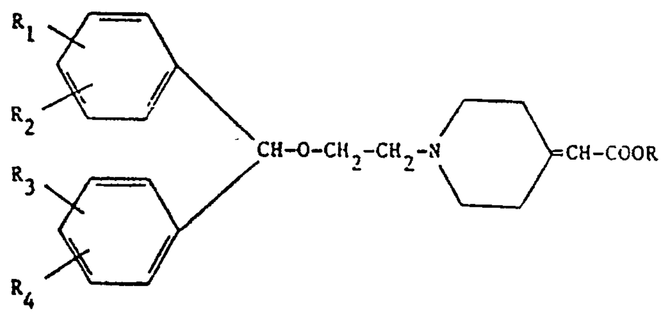

(II)

dans lesquelles :

- $R_1$, $R_2$, $R_3$ et $R_4$, identiques ou différents, représentent un atome d'hydrogène, un atome d'halogène, un groupe alkyle ayant de 1 à 6 atomes de carbone, un groupe alcoxy ayant de 1 à 6 atomes de carbone ou le groupe trifluorométhyle,
- R représente l'hydrogène, un cation, un groupe alkyle ayant de 1 à 6 atomes de carbone,
- n est égal à 0 ou 1 et leurs sels pharmaceutiquement acceptables.

**2.** Dérivés selon la revendication 1, caractérisé en ce qu'ils sont :

- le 1-(benzhydryloxyéthyl)pipéridino-4-acétate d'éthyle,
- le 1-(benzhydryloxyéthyl)pipérid-4-ylidène acétate d'éthyle,
- le 4-carbéthoxy-1-benzhydryloxyéthyl-pipéridine et leurs sels pharmaceutiquement acceptables.

**3.** Procédé pour l'obtention des dérivés de formule I selon la revendication 1, caractérisé en ce qu'il consiste :

à chauffer à reflux un dérivé halogéné de formule III

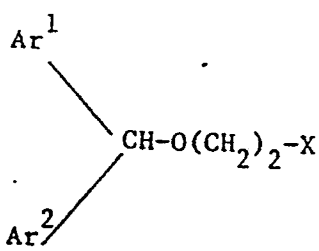

(III)

dans laquelle $Ar^1$ et $Ar^2$ représentent respectivement les groupes de formule :

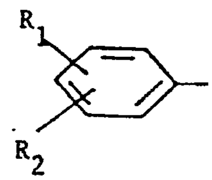

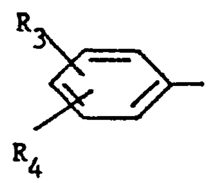

dans lesquelles $R_1$, $R_2$, $R_3$ et $R_4$ sont tels que définis dans la revendication 1 et X est un atome d'hologène, avec une amine de formule IV :

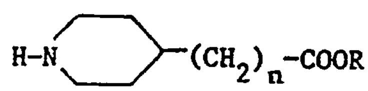

(IV)

dans laquelle n est égal à 0 ou 1 et R est tel que défini dans la revendication 1, dans un solvant aromatique et en présence d'un agent accepteur d'acide.

4. Procédé pour l'obtention des dérivés de formule I selon la revendication 1, caractérisé en ce qu'il consiste à soumettre à une hydrogénation catalytique en présence de nickel de Raney, à température ambiante et sous pression d'hydrogène, le dérivé éthylènique correspondant à la formule II (n = 1) dans un alcool primaire.

5. Procédé pour l'obtention des dérivés de formule II selon la revendication 1, caractérisé en ce qu'il consiste à faire réagir dans le tétrahydrofuranne à 0 ° C, le carbanion sodé du diéthylphosphonoacétate d'alkyle de formule :

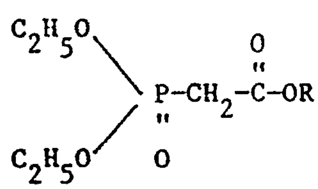

dans laquelle R est tel que défini dans la revendication 3, sur la benzhydryloxyéthylpipéridone de formule VII,

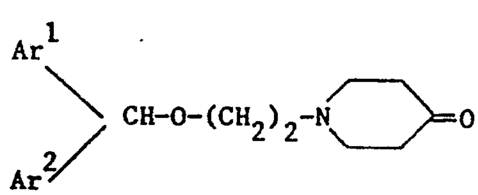

dans laquelle $Ar^1$ et $Ar^2$ sont tels que définis dans la revendication 3.

6. Compositions pharmaceutiques caractérisées en ce qu'elles contiennent, à titre d'ingrédient actif, un dérivé selon l'une des revendications 1 ou 2, en combinaison avec un véhicule pharmaceutiquemnt

acceptable.

**Revendications pour les Etats contractants suivants : AT, ES**

**1.** Procédé pour l'obtention de dérivés de la benzhydryloxyéthyl-pipéridine répondant à la formule :

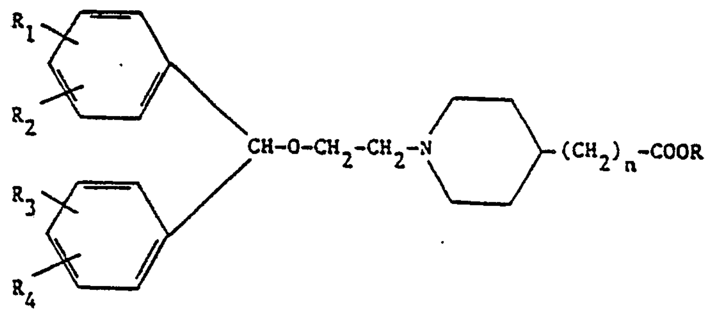

(I)

dans laquelle :

- $R_1$, $R_2$, $R_3$ et $R_4$, identiques ou différents, représentent un atome d'hydrogène, un atome d'halogène, un groupe alkyle ayant de 1 à 6 atomes de carbone, un groupe alcoxy ayant de 1 à 6 atomes de carbone ou le groupe trifluorométhyle,
- R représente l'hydrogène, un cation, un groupe alkyle ayant de 1 à 6 atomes de carbone,
- n est égal à 0 ou 1 et leurs sels pharmaceutiquement acceptables,

caractérisé en ce qu'il consiste à chauffer à reflux un dérivé halogéné de formule III

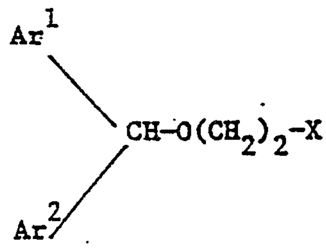

(III)

dans laquelle $Ar^1$ et $Ar^2$ représentent respectivement les groupes de formule

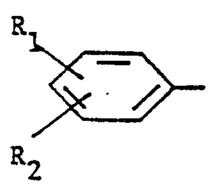

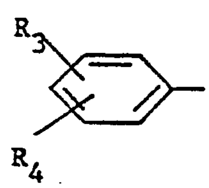

dans lesquelles $R_1$, $R_2$, $R_3$ et $R_4$ sont tels que définis précédemment et X est un atome d'halogène, avec une amine de formule IV

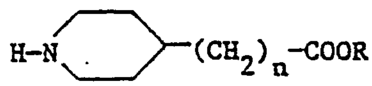

(IV)

dans laquelle n et R sont tels que définis ci-dessus, dans un solvant aromatique et en présence d'un agent accepteur d'acide et, à transformer éventuellement le composé ainsi obtenu en l'un de ses sels pharmaceutiquement acceptables.

**2.** Procédé pour l'obtention des dérivés de formule I tels que définis dans la revendication 1 avec n = 1, caractérisé en ce qu'il consiste à soumettre à une hydrogénation catalytique en présence de nickel de Raney, à température ambiante et sous pression d'hydrogène, le dérivé éthylénique correspondant de formule II (n = 1) dans un alcool primaire et, à transformer éventuellement le composé ainsi obtenu en l'un de ses sels pharmaceutiquement acceptables.

**3.** Procédé pour l'obtention de dérivés de la benzhydryloxyéthyl-pipéridine répondant à la formule :

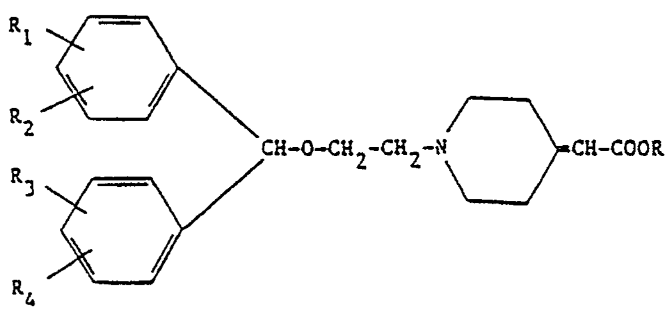

(II)

dans laquelle :

- $R_1$, $R_2$, $R_3$ et $R_4$, identiques ou différents, représentent un atome d'hydrogène, un atome d'halogène, un groupe alkyle ayant de 1 à 6 atomes de carbone, un groupe alcoxy ayant de 1 à 6 atomes de carbone ou le groupe trifluorométhyle.
- R représente l'hydrogène, un cation, un groupe alkyle ayant de 1 à 6 atomes de carbone.

et leurs sels pharmaceutiquement acceptables, caractérisé en ce qu'il consiste à faire réagir dans le tétrahydrofuranne à 0 °C, le carbanion sodé du diéthylphosphonoacétate d'alkyle de formule :

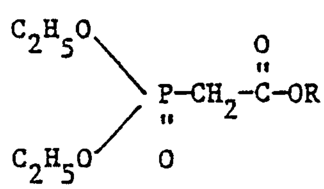

dans laquelle R est tel que défini ci-dessus, sur la benzhydryloxyéthylpipéridone de formule VII,

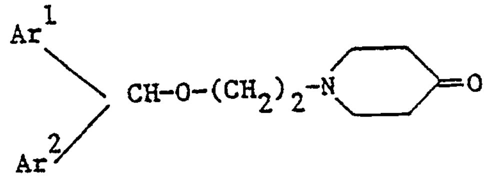

dans laquelle $Ar^1$ et $Ar^2$ sont tels que définis dans la revendication 1 et, à transformer éventuellement le composé ainsi obtenu en l'un de ses sels pharmaceutiquement

acceptables.

4. Utilisation des dérivés de la benzhydryloxyéthyl-pipéridine répondant à l'une des formules ci-après :

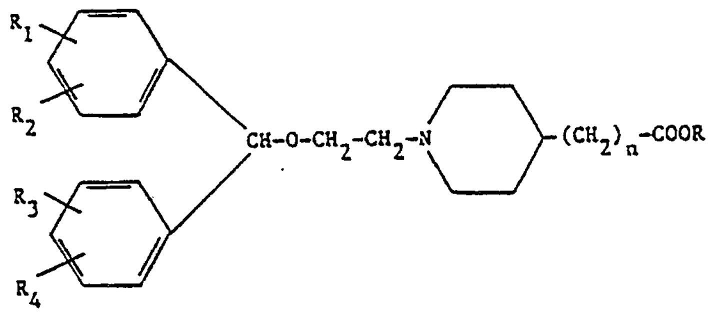

(I)

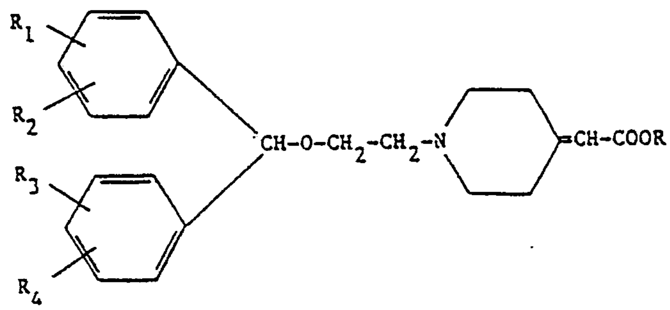

(II)

- $R_1$, $R_2$, $R_3$ et $R_4$, identiques ou différents, représentent un atome d'hydrogène, un atome d'halogène, un groupe alkyle ayant de 1 à 6 atomes de carbone, un groupe alcoxy ayant de 1 à 6 atomes de carbone ou le groupe trifluorométhyle,
- R représente l'hydrogène, un cation, un groupe alkyle ayant de 1 à 6 atomes de carbone,
- n est égal à 0 ou 1 et leurs sels pharmaceutiquement acceptables,

pour la préparation de compositions pharmaceutiques utiles notamment pour le traitement des états spasmodiques et des allergies.

**Revendications pour l'Etat contractant suivant : GR**

1. Dérivés de benzhydryloxyéthyl-pipéridine caractérisés en ce qu'ils répondent à l'une des formules ci-après :

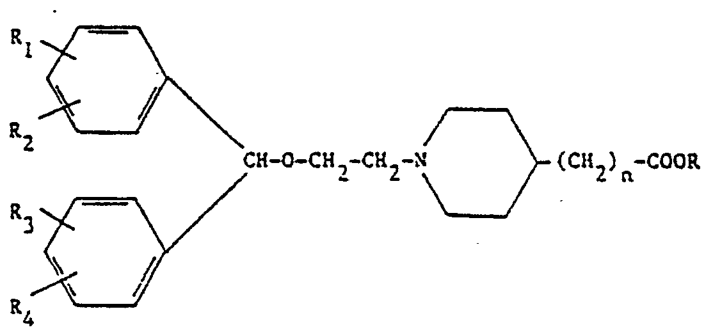

(I)

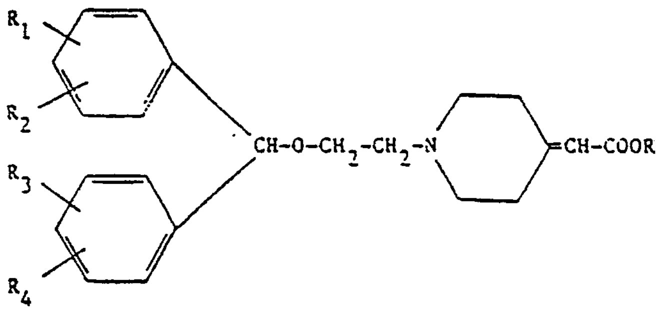

(II)

dans lesquelles :

- $R_1$, $R_2$, $R_3$ et $R_4$, identiques ou différents, représentent un atome d'hydrogène, un atome d'halogène, un groupe alkyle ayant de 1 à 6 atomes de carbone, un groupe alcoxy ayant de 1 à 6 atomes de carbone ou le groupe trifluorométhyle,
- R représente l'hydrogène, un cation, un groupe alkyle ayant de 1 à 6 atomes de carbone,
- n est égal à 0 ou 1 et leurs sels pharmaceutiquement acceptables.

**2.** Dérivés selon la revendication 1, caractérisé en ce qu'ils sont :

- le 1-(benzhydryloxyéthyl)pipéridino-4-acétate d'éthyle,
- le 1-(benzhydryloxyéthyl)pipérid-4-ylidène acétate d'éthyle,
- le 4-carbéthoxy-1-benzhydryloxyéthyl-pipéridine et leurs sels pharmaceutiquement acceptables.

**3.** Procédé pour l'obtention des dérivés de formule I selon la revendication 1, caractérisé en ce qu'il consiste :

à chauffer à reflux un dérivé halogéné de formule III

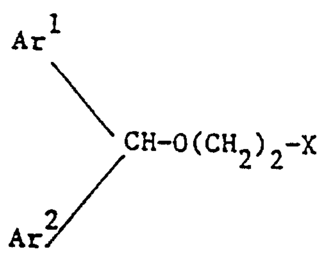

(III)

dans laquelle $Ar^1$ et $Ar^2$ représentent respectivement les groupes de formule :

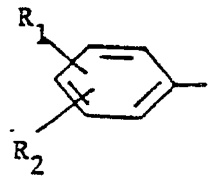

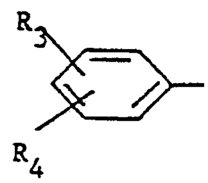

dans lesquelles $R_1$, $R_2$, $R_3$ et $R_4$ sont tels que définis dans la revendication 1 et X est un atome d'hologène, avec une amine de formule IV :

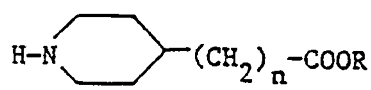

(IV)

dans laquelle n est égal à 0 ou 1 et R est tel que défini dans la revendication 1, dans un solvant aromatique et en présence d'un agent accepteur d'acide.

4. Procédé pour l'obtention des dérivés de formule I selon la revendication 1, caractérisé en ce qu'il consiste à soumettre à une hydrogénation catalytique en présence de nickel de Raney, à température ambiante et sous pression d'hydrogène, le dérivé éthylènique correspondant à la formule II (n = 1) dans un alcool primaire.

5. Procédé pour l'obtention des dérivés de formule II selon la revendication 1, caractérisé en ce qu'il consiste à faire réagir dans le tétrahydrofuranne à 0 ° C, le carbanion sodé du diéthylphosphonoacétate d'alkyle de formule :

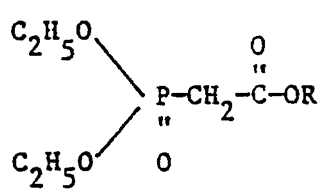

dans laquelle R est tel que défini dans la revendication 3, sur la benzhydryloxyéthylpipéridone de formule VII,

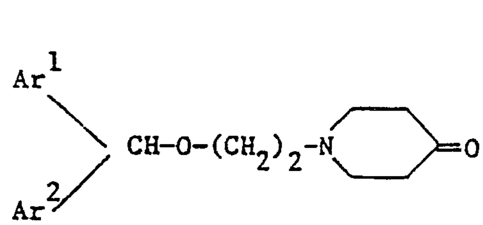

dans laquelle $Ar^1$ et $Ar^2$ sont tels que définis dans la revendication 3.

6. Utilisation des dérivés de la benzhydryloxyéthyl-pipéridine répondant à l'une des formules ci-après :

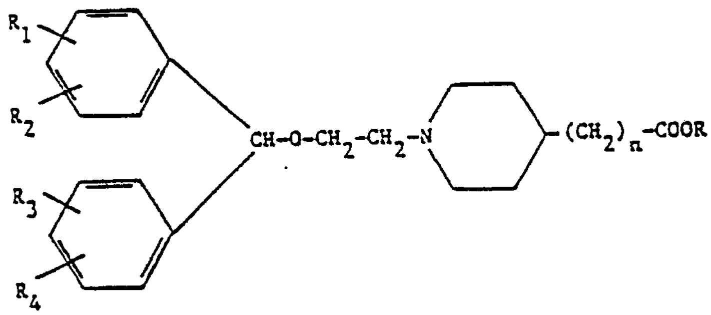

(I)

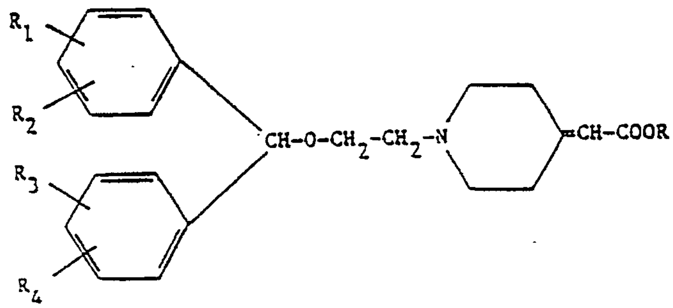

(II)

- $R_1$, $R_2$, $R_3$ et $R_4$, identiques ou différents, représentent un atome d'hydrogène, un atome d'halogène, un groupe alkyle ayant de 1 à 6 atomes de carbone, un groupe alcoxy ayant de 1 à 6 atomes de carbone ou le groupe trifluorométhyle,
- R représente l'hydrogène, un cation, un groupe alkyle ayant de 1 à 6 atomes de carbone,
- n est égal à 0 ou 1 et leurs sels pharmaceutiquement acceptables,

pour la préparation de compositions pharmaceutiques utiles notamment pour le traitement des états spasmodiques et des allergies.

**Claims**

**Claims for the following Contracting States : BE, CH, DE, FR, GB, IT, LI, LU, NL, SE**

**1.** Benzhydryloxyethylpiperidine derivatives characterized in that they correspond to one of the formulae below:

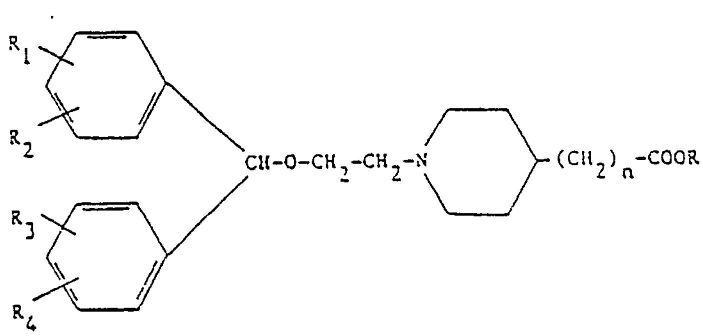

(I)

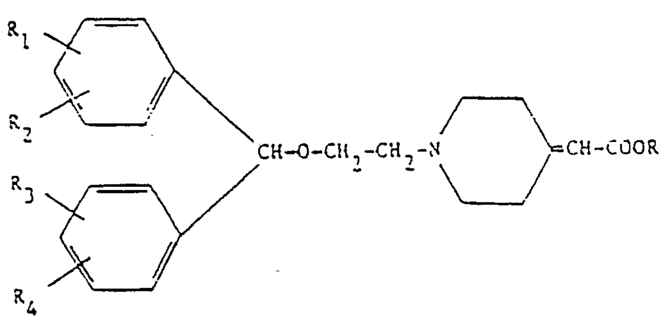

(II)

in which:

- $R_1$, $R_2$, $R_3$ and $R_4$, which are identical or different, represent a hydrogen atom, a halogen atom, an alkyl group containing from 1 to 6 carbon atoms, an alkoxy group containing from 1 to 6 carbon atoms or the trifluoromethyl group,
- R represents hydrogen, a cation, an alkyl group containing from 1 to 6 carbon atoms,
- n is equal to 0 or 1,

and their pharmaceutically acceptable salts.

**2.** The derivatives according to claim 1, characterized in that they are:

- ethyl 1-(benzhydryloxyethyl)piperidino-4-acetate,
- ethyl 1-(benzhydryloxyethyl)piperidin-4-ylideneacetate and
- 4-carbethoxy-1-benzhydryloxyethylpiperidine

and their pharmaceutically acceptable salts.

**3.** A process for the preparation of the derivatives of the formula (I) according to claim 1, characterized in that it consists:
in refluxing a halogen derivative of the formula (III):

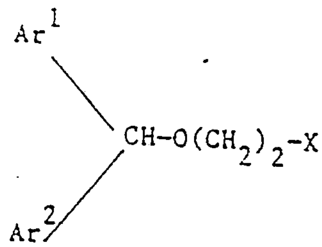

(III)

in which $Ar^1$ and $Ar^2$ respectively represent the groups of the formulae:

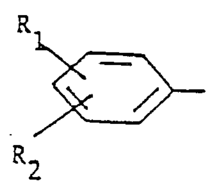

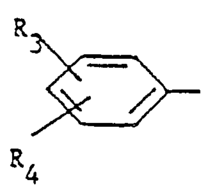

in which $R_1$, $R_2$, $R_3$ and $R_4$ are as defined in claim 1 and X is a halogen atom, with an amine of the formula (IV):

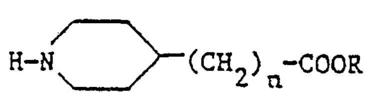

(IV)

in which n is equal to 0 or 1, and R is as defined in claim 1, in an aromatic solvent and in the presence of an acid acceptor.

**4.** A process for the preparation of the derivatives of the formula (I) according to claim 1, characterized in that it consists in subjecting the corresponding ethylenic derivative of the formula (II) (n = 1) to

catalytic hydrogenation in a primary alcohol, in the presence of Raney nickel, at room temperature and under hydrogen pressure.

5. A process for the preparation of the derivatives of the formula (II) according to claim 1, characterized in that it consists in reacting the sodium carbanion of an alkyl diethylphosphonoacetate of the formula:

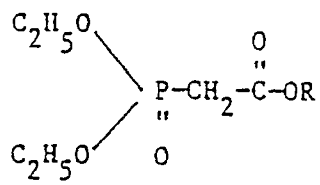

in which R is as defined in claim 3, with a benzhydryloxyethylpiperidone of the formula (VII):

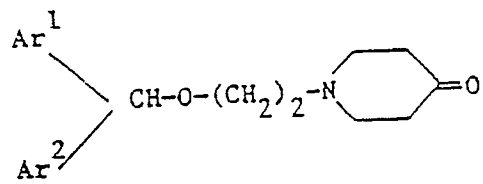

in which $Ar^1$ and $Ar^2$ are as defined in claim 3, in tetrahydrofuran at 0° C.

6. Pharmaceutical compositions characterized in that they contain a derivative according to one of claims 1 or 2 as the active ingredient, in combination with a pharmaceutically acceptable vehicle.

**Claims for the following Contracting States : AT, ES**

1. A process for the preparation of benzhydryloxyethylpiperidine derivatives corresponding to the formula:

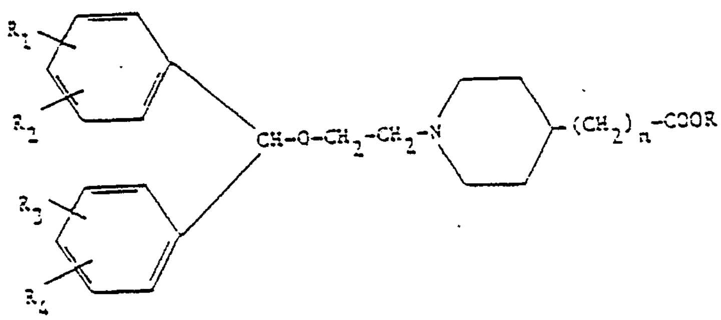

(I)

in which:
- $R_1$, $R_2$, $R_3$ and $R_4$, which are identical or different, represent a hydrogen atom, a halogen atom, an alkyl group containing from 1 to 6 carbon atoms, an alkoxy group containing from 1 to 6 carbon atoms or the trifluoromethyl group,
- R represents hydrogen, a cation, an alkyl group containing from 1 to 6 carbon atoms,
- n is equal to 0 or 1,

and their pharmaceutically acceptable salts,
characterized in that it consists in refluxing a halogen derivative of the formula (III):

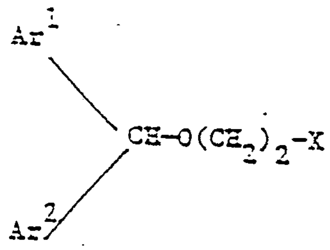

(III)

in which $Ar^1$ and $Ar^2$ respectively represent the groups of the formulae:

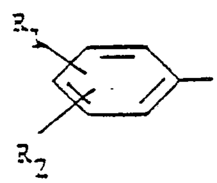

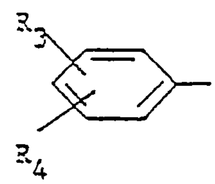

in which $R_1$, $R_2$, $R_3$ and $R_4$ are as defined above and X is a halogen atom, with an amine of the formula (IV):

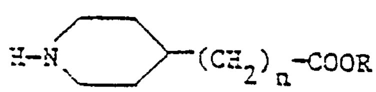

(IV)

in which n and R are as defined above in an aromatic solvent and in the presence of an acid acceptor and, in converting, if appropriate, the resulting compound to one of its pharmaceutically acceptable salts.

2. A process for the preparation of the derivatives of the formula (I) such as defined in claim 1, with n = 1, characterized in that it consists in subjecting the corresponding ethylenic derivative of the formula (II) (n = 1) to a catalytic hydrogenation in a primary alcohol, in the presence of Raney nickel, at room temperature and under hydrogen pressure, and in converting, if appropriate, the resulting compound to one of its pharmaceutically acceptable salts.

3. A process for the preparation of benzhydryloxyethylpiperidine derivatives corresponding to the formula:

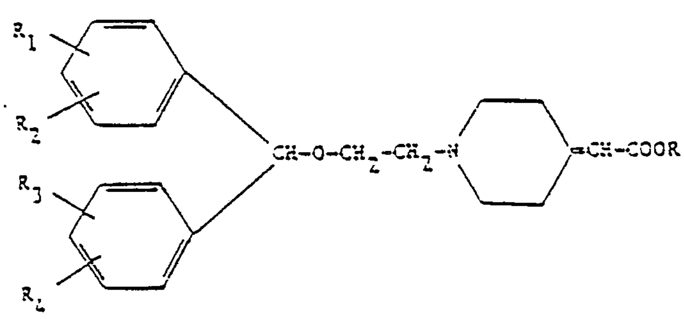

(II)

in which:

- $R_1$, $R_2$, $R_3$ and $R_4$, which are identical or different, represent a hydrogen atom, a halogen atom, an alkyl group containing from 1 to 6 carbon atoms, an alkoxy group containing from 1 to 6 carbon atoms or the trifluoromethyl group,
- R represents hydrogen, a cation, an alkyl group containing from 1 to 6 carbon atoms,

and their pharmaceutically acceptable salts, characterized in that it consists in reacting the sodium carbanion of an alkyl diethylphosphonoacetate of the formula:

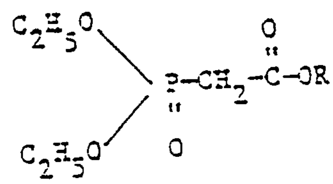

in which R is as defined above, with a benzhydryloxyethylpiperidone of the formula (VII):

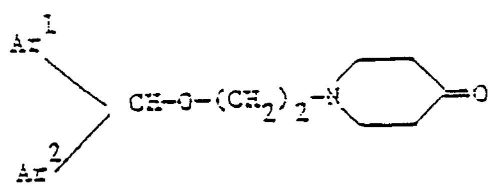

in which $Ar^1$ and $Ar^2$ are as defined in claim 1, and in converting, if appropriate, the compound obtained to one of its pharmaceutically acceptable salts.

4. Use of the benzhydryloxyethylpiperidine derivatives corresponding to one of the formulae below:

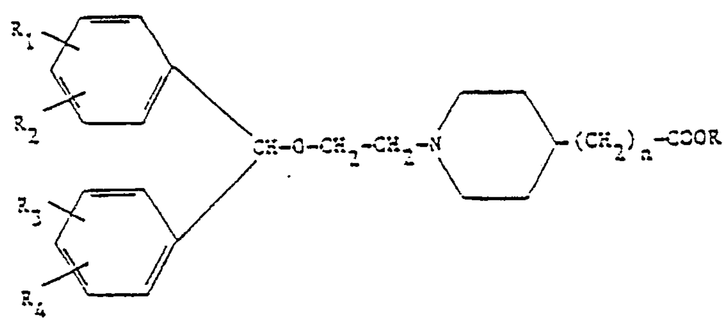

(I)

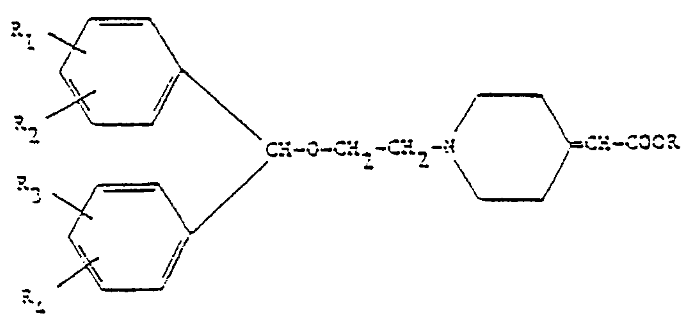

(II)

- $R_1$, $R_2$, $R_3$ and $R_4$, which are identical or different, represent a hydrogen atom, a halogen atom, an alkyl group containing from 1 to 6 carbon atoms, an alkoxy group containing from 1 to 6 carbon atoms or the trifluoromethyl group,
- R represents hydrogen, a cation, an alkyl group containing from 1 to 6 carbon atoms,

- n is equal to 0 or 1,

and their pharmaceutically acceptable salts, for the preparation of pharmaceutical compositions useful especially for the treatment of spasmodic states and allergies.

**Claims for the following Contracting State : GR**

**1.** Benzhydryloxyethylpiperidine derivatives characterized in that they correspond to one of the formulae below:

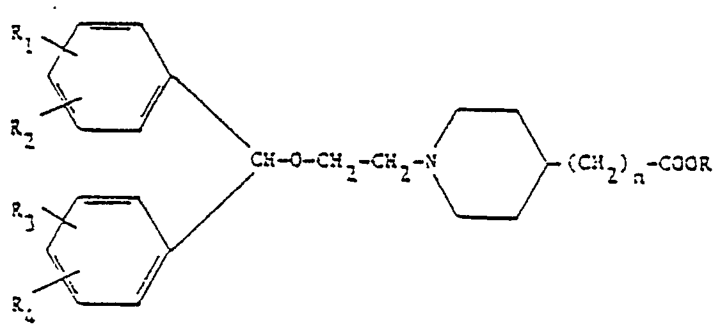
(I)

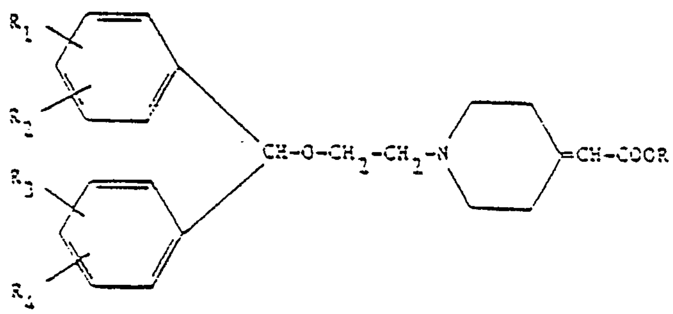
(II)

in which:
- $R_1$, $R_2$, $R_3$ and $R_4$, which are identical or different, represent a hydrogen atom, a halogen atom, an alkyl group containing from 1 to 6 carbon atoms, an alkoxy group containing from 1 to 6 carbon atoms or the trifluoromethyl group,
- R represents hydrogen, a cation, an alkyl group containing from 1 to 6 carbon atoms,
- n is equal to 0 or 1,

and their pharmaceutically acceptable salts.

**2.** The derivatives according to claim 1, characterized in that they are:
- ethyl 1-(benzhydryloxyethyl)piperidino-4-acetate,
- ethyl 1-(benzhydryloxyethyl)piperidin-4-ylideneacetate and
- 4-carbethoxy-1-benzhydryloxyethylpiperidine

and their pharmaceutically acceptable salts.

**3.** A process for the preparation of the derivatives of the formula (I) according to claim 1, characterized in that it consists:
in refluxing a halogen derivative of the formula (III):

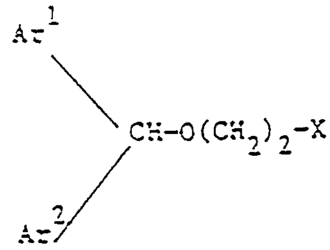

(III)

in which $Ar^1$ and $Ar^2$ respectively represent the groups of the formulae:

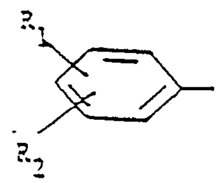

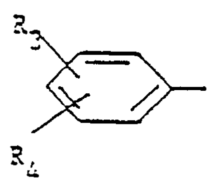

in which $R_1$, $R_2$, $R_3$ and $R_4$ are as defined in claim 1 and X is a halogen atom, with an amine of the formula (IV):

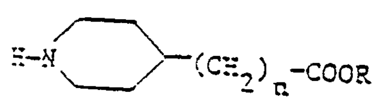

(IV)

in which n is equal to 0 or 1, and R is as defined in claim 1, in an aromatic solvent and in the presence of an acid acceptor.

4. A process for the preparation of the derivatives of the formula (I) according to claim 1, characterized in that it consists in subjecting the corresponding ethylenic derivative of the formula (II) (n = 1) to catalytic hydrogenation in a primary alcohol, in the presence of Raney nickel, at room temperature and under hydrogen pressure.

5. A process for the preparation of the derivatives of the formula (II) according to claim 1, characterized in that it consists in reacting the sodium carbanion of an alkyl diethylphosphonoacetate of the formula:

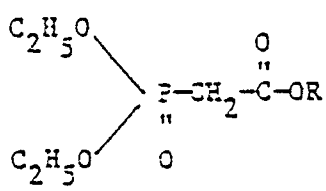

in which R is as defined in claim 3, with a benzhydryloxyethylpiperidone of the formula (VII):

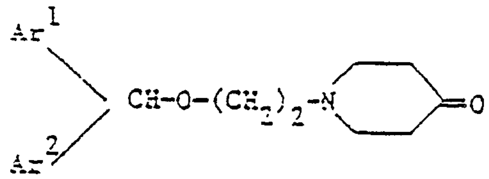

in which $Ar^1$ and $Ar^2$ are as defined in claim 3, in tetrahydrofuran at 0° C.

6. Use of the benzhydryloxyethylpiperidine corresponding to one of the formulae below:

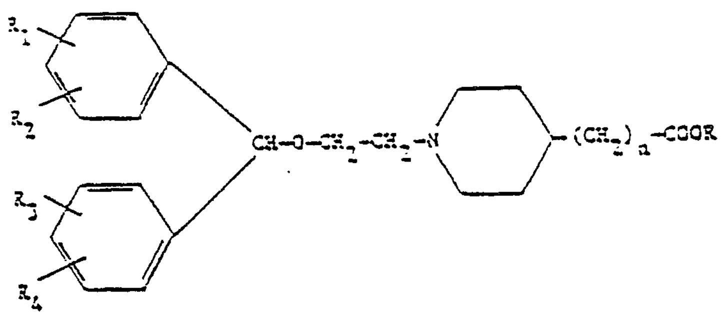

(I)

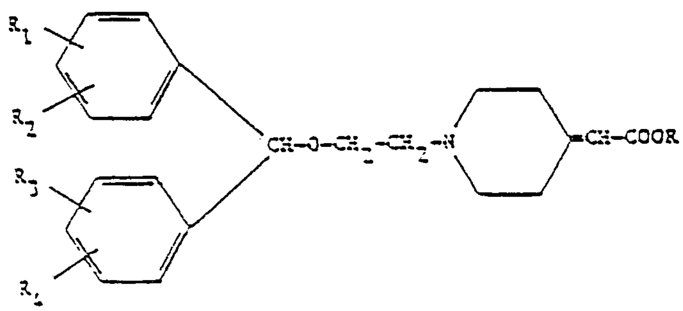

(II)

- $R_1$, $R_2$, $R_3$ and $R_4$, which are identical or different, represent a hydrogen atom, a halogen atom, an alkyl group containing from 1 to 6 carbon atoms, an alkoxy group containing from 1 to 6 carbon atoms or the trifluoromethyl group,
- R represents hydrogen, a cation, an alkyl group containing from 1 to 6 carbon atoms,
- n is equal to 0 or 1,

and their pharmaceutically acceptable salts, for the preparation of pharmaceutical compositions useful especially for the treatment of spasmodic states and allergies.

**Patentansprüche**

**Patentansprüche für folgende Vertragsstaaten : BE, CH, DE, FR, GB, IT, LI, LU, NL, SE**

1. Benzhydryloxyethyl-piperidinderivate, dadurch gekennzeichnet, daß sie einer der nachstehenden Formeln entsprechen:

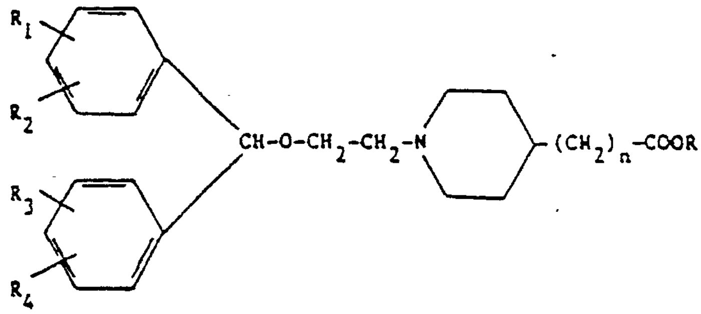 (I)

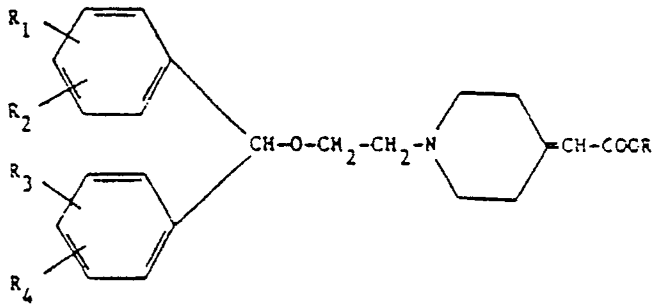 (II)

in denen:

- $R_1$, $R_2$, $R_3$ und $R_4$, gleich oder verschieden, ein Wasserstoffatom, ein Halogenatom, eine Alkylgruppe mit 1 bis 6 Kohlenstoffatomen, eine Alkoxygruppe mit 1 bis 6 Kohlenstoffatomen oder Trifluormethyl darstellen,
- R Wasserstoff, ein Kation, eine Alkylgruppe mit 1 bis 6 Kohlenstoffatomen darstellt,
- n für 0 oder 1 steht,

und ihre pharmazeutisch akzeptablen Salze.

**2.** Derivate nach Anspruch 1, dadurch gekennzeichnet, daß sie sind:

- 1-(Benzhydryloxyethyl)-piperidino-4-ethylacetat
- 1-(Benzhydryloxyethyl)-piperid-4-yliden-ethylacetat
- 4-Carbethoxy-1-benzhydryloxyethyl-piperidin

und ihre pharmazeutisch akzeptablen Salze.

**3.** Verfahren zur Herstellung der Derivate der Formel I nach Anspruch 1, dadurch gekennzeichnet, daß es darin besteht, daß

ein Halogenderivat der Formel III

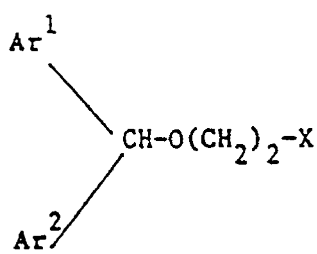 (III)

worin $Ar^1$ und $Ar^2$ die Gruppen der Formel

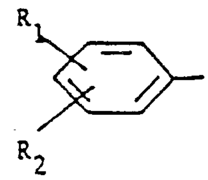

bzw.

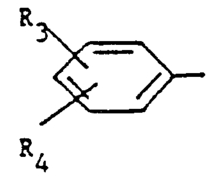

darstellen, worin $R_1$, $R_2$, $R_3$ und $R_4$ wie in Anspruch 1 definiert sind und X ein Halogenatom ist, mit einem Amin der Formel IV

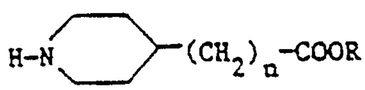

(IV)

worin n für 0 oder 1 steht und R wie in Anspruch 1 definiert ist, in einem aromatischen Lösungsmittel und in Gegenwart eines Säureakzeptors auf Rückfluß erhitzt wird.

4. Verfahren zur Herstellung der Derivate der Formel I nach Anspruch 1, dadurch gekennzeichnet, daß es darin besteht, daß das der Formel II (n = 1) entsprechende Ethylenderivat in einem primären Alkohol einer katalytischen Hydrierung in Gegenwart von Raney-Nickel bei Umgebungstemperatur und unter Wasserstoffdruck unterzogen wird.

5. Verfahren zur Herstellung der Derivate der Formel II nach Anspruch 1, dadurch gekennzeichnet, daß es darin besteht, daß das Natriumcarbanion von Alkyl-diethylphosphonoacetat der Formel

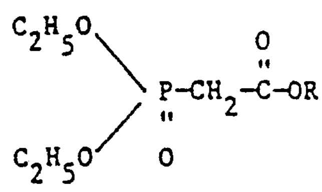

worin R wie in Anspruch 3 definiert ist, mit Benzhydryloxyethylpiperidon der Formel VII

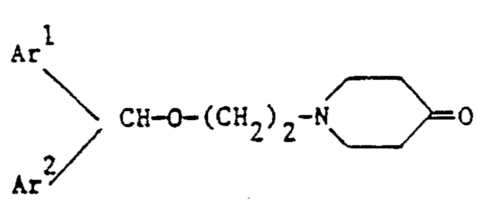

worin $Ar^1$ und $Ar^2$ wie in Anspruch 3 definiert sind, in Tetrahydrofuran bei 0 °C reagieren gelassen wird.

6. Pharmazeutische Zusammensetzungen, dadurch gekennzeichnet, daß sie als aktives Ingrediens ein Derivat nach einem der Ansprüche 1 oder 2 in Kombination mit einem pharmazeutisch akzeptablen Vehikel enthalten.

**Patentansprüche für folgende Vertragsstaaten : AT, ES**

1. Verfahren zur Herstellung von Benzhydryloxyethylpiperidinderivaten der Formel:

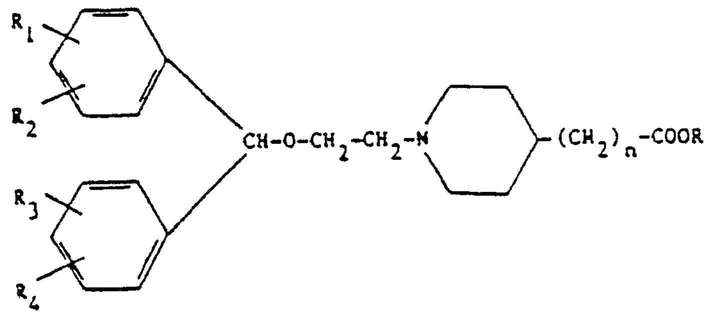

(I)

in denen:

- $R_1$, $R_2$, $R_3$ und $R_4$, gleich oder verschieden, ein Wasserstoffatom, ein Halogenatom, eine Alkylgruppe mit 1 bis 6 Kohlenstoffatomen, eine Alkoxygruppe mit 1 bis 6 Kohlenstoffatomen oder Trifluormethyl darstellen,
- R Wasserstoff, ein Kation, eine Alkylgruppe mit 1 bis 6 Kohlenstoffatomen darstellt,
- n für 0 oder 1 steht,

und ihren pharmazeutisch akzeptablen Salzen,
dadurch gekennzeichnet, daß es darin besteht, daß
ein Halogenderivat der Formel III

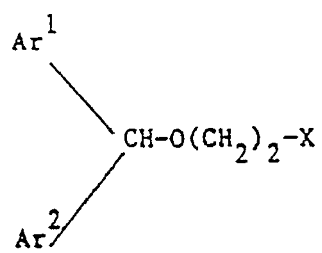

(III)

worin $Ar^1$ und $Ar^2$ die Gruppen der Formel

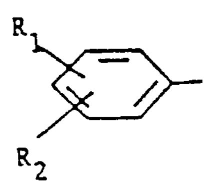

bzw.

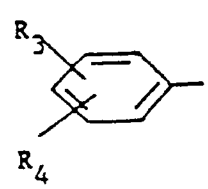

darstellen, in denen $R_1$, $R_2$, $R_3$ und $R_4$ wie zuvor definiert sind und X ein Halogenatom ist, mit einem Amin der Formel IV

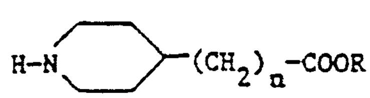

(IV)

worin n für 0 oder 1 steht und R wie oben definiert ist, in einem aromatischen Lösungsmittel und in Gegenwart eines Säureakzeptors auf Rückfluß erhitzt wird und gegebenenfalls die so erhaltene

Verbindung in eines ihrer pharmazeutisch akzeptablen Salze übergeführt wird.

2. Verfahren zur Herstellung der Derivate der Formel I nach Anspruch 1 mit n = 1, dadurch gekennzeichnet, daß es darin besteht, daß das der Formel II (n = 1) entsprechende Ethylenderivat in einem primären Alkohol einer katalytischen Hydrierung in Gegenwart von Raney-Nickel bei Umgebungstemperatur und unter Wasserstoffdruck unterzogen wird und gegebenenfalls die so erhaltene Verbindung in eines ihrer pharmazeutisch akzeptablen Salze übergeführt wird.

3. Verfahren zur Herstellung von Benzhydryloxyethylpiperidinderivaten der Formel:

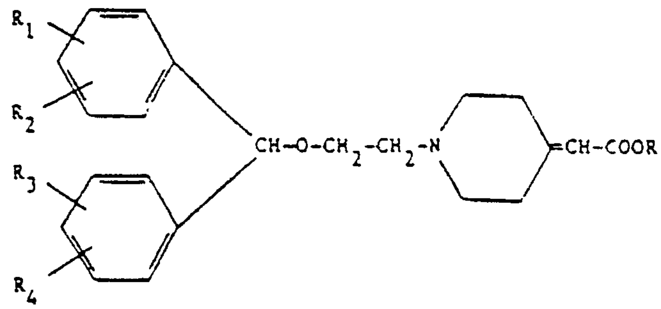

(II)

in denen:

- $R_1$, $R_2$, $R_3$ und $R_4$, gleich oder verschieden, ein Wasserstoffatom, ein Halogenatom, eine Alkylgruppe mit 1 bis 6 Kohlenstoffatomen, eine Alkoxygruppe mit 1 bis 6 Kohlenstoffatomen oder Trifluormethyl darstellen,
- R Wasserstoff, ein Kation, eine Alkylgruppe mit 1 bis 6 Kohlenstoffatomen darstellt,

und ihren pharmazeutisch akzeptablen Salzen, dadurch gekennzeichnet, daß es darin besteht, daß das Natriumcarbanion von Alkyl-diethylphosphonoacetat der Formel

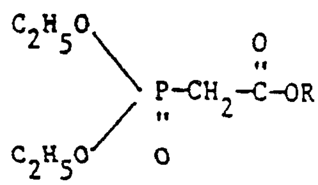

worin R wie oben definiert ist, mit Benzhydryloxyethylpiperidon der Formel VII

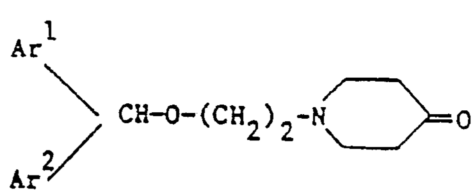

worin $Ar^1$ und $Ar^2$ wie in Anspruch 1 definiert sind, in Tetrahydrofuran bei 0 °C reagieren gelassen wird und gegebenenfalls die so erhaltene Verbindung in eines ihrer pharmazeutisch akzeptablen Salze übergeführt wird.

4. Verwendung der Benzhydryloxyethyl-piperidinderivate einer der nachstehenden Formeln

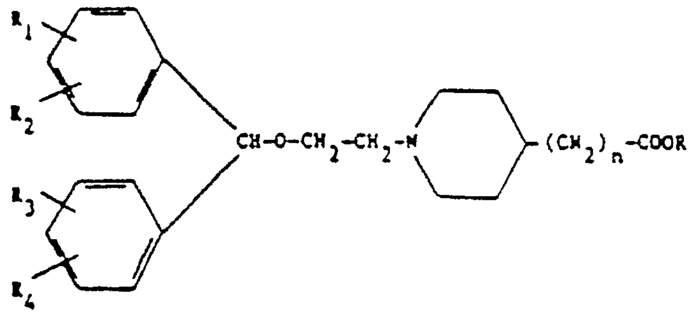 (I)

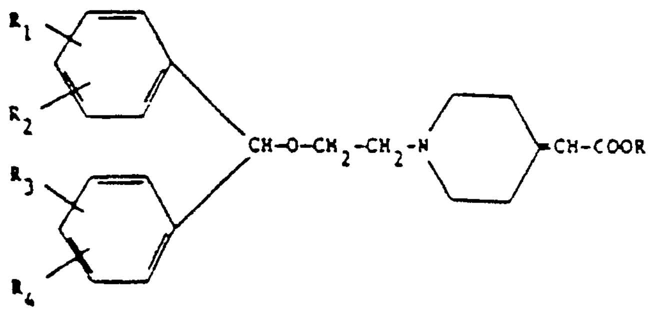 (II)

in denen:

- $R_1$, $R_2$, $R_3$ und $R_4$, gleich oder verschieden, ein Wasserstoffatom, ein Halogenatom, eine Alkylgruppe mit 1 bis 6 Kohlenstoffatomen, eine Alkoxygruppe mit 1 bis 6 Kohlenstoffatomen oder Trifluormethyl darstellen,
- R Wasserstoff, ein Kation, eine Alkylgruppe mit 1 bis 6 Kohlenstoffatomen darstellt,
- n für 0 oder 1 steht,

und ihrerpharmazeutisch akzeptablen Salze zur Herstellung von pharmazeutischen Zusammensetzungen, die insbesondere zur Behandlung von Krampfzuständen und Allergien nützlich sind.

**Patentansprüche für folgenden Vertragsstaat : GR**

1. Benzhydryloxyethyl-piperidinderivate, dadurch gekennzeichnet, daß sie einer der nachstehenden Formeln entsprechen:

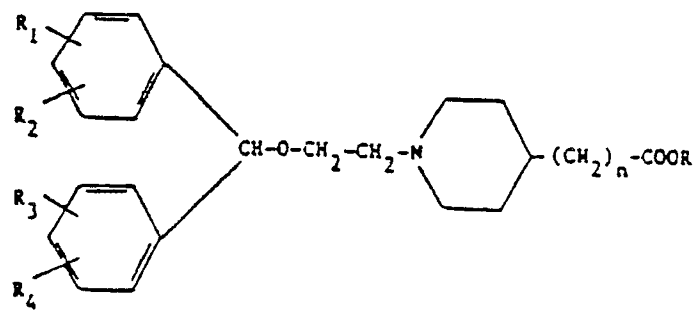

(I)

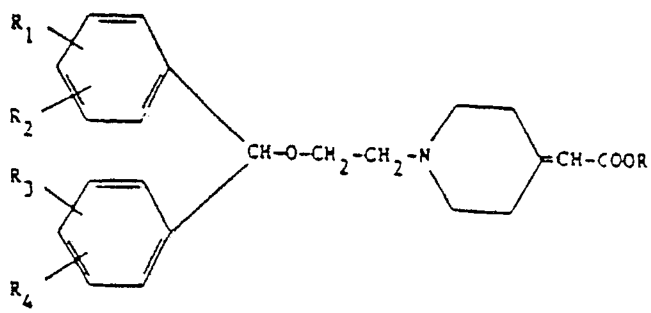

(II)

in denen:

- $R_1$, $R_2$, $R_3$ und $R_4$, gleich oder verschieden, ein Wasserstoffatom, ein Halogenatom, eine Alkylgruppe mit 1 bis 6 Kohlenstoffatomen, eine Alkoxygruppe mit 1 bis 6 Kohlenstoffatomen oder Trifluormethyl darstellen,
- R Wasserstoff, ein Kation, eine Alkylgruppe mit 1 bis 6 Kohlenstoffatomen darstellt,
- n für 0 oder 1 steht,

und ihre pharmazeutisch akzeptablen Salze.

**2.** Derivate nach Anspruch 1, dadurch gekennzeichnet, daß sie sind:

- 1-(Benzhydryloxyethyl)-piperidino-4-ethylacetat
- 1-(Benzhydryloxyethyl)-piperid-4-yliden-ethylacetat
- 4-Carbethoxy-1-benzhydryloxyethyl-piperidin

und ihre pharmazeutisch akzeptablen Salze.

**3.** Verfahren zur Herstellung der Derivate der Formel I nach Anspruch 1, dadurch gekennzeichnet, daß es darin besteht, daß

ein Halogenderivat der Formel III

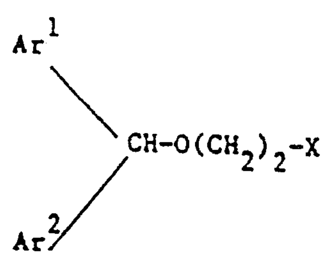

(III)

worin $Ar^1$ und $Ar^2$ die Gruppen der Formel

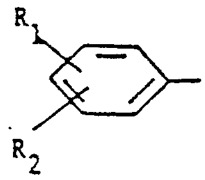

bzw.

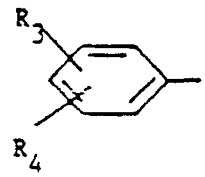

darstellen, worin $R_1$, $R_2$, $R_3$ und $R_4$ wie in Anspruch 1 definiert sind und X ein Halogenatom ist, mit einem Amin der Formel IV

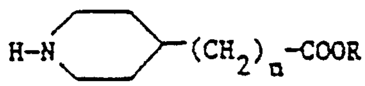

(IV)

worin n für 0 oder 1 steht und R wie in Anspruch 1 definiert ist, in einem aromatischen Lösungsmittel und in Gegenwart eines Säureakzeptors auf Rückfluß erhitzt wird.

4. Verfahren zur Herstellung der Derivate der Formel I nach Anspruch 1, dadurch gekennzeichnet, daß es darin besteht, daß das der Formel II (n = 1) entsprechende Ethylenderivat in einem primären Alkohol einer katalytischen Hydrierung in Gegenwart von Raney-Nickel bei Umgebungstemperatur und unter Wasserstoffdruck unterzogen wird.

5. Verfahren zur Herstellung der Derivate der Formel II nach Anspruch 1, dadurch gekennzeichnet, daß es darin besteht, daß das Natriumcarbanion von Alkyl-diethylphosphonoacetat der Formel

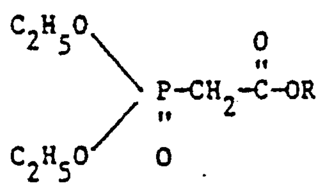

worin R wie in Anspruch 3 definiert ist, mit Benzhydryloxyethylpiperidon der Formel VII

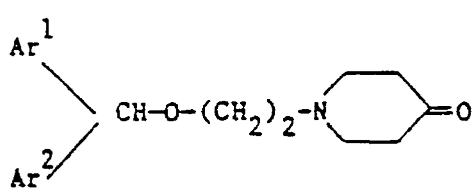

worin $Ar^1$ und $Ar^2$ wie in Anspruch 3 definiert sind, in Tetrahydrofuran bei 0 °C reagieren gelassen wird.

6. Verwendung der Benzhydryloxyethyl-piperidinderivate einer der nachstehenden Formeln

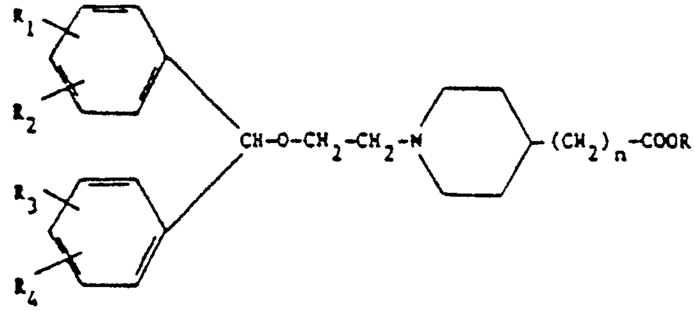 (I)

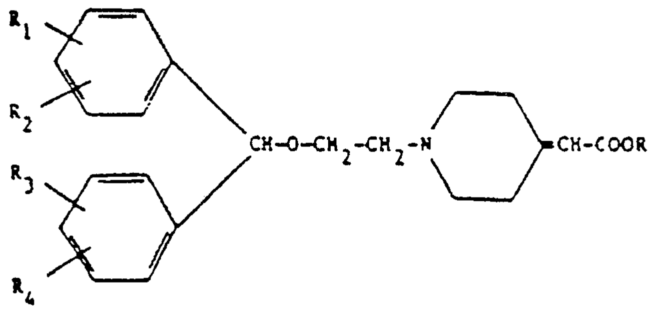 (II)

in denen:

- $R_1$, $R_2$, $R_3$ und $R_4$, gleich oder verschieden, ein Wasserstoffatom, ein Halogenatom, eine Alkylgruppe mit 1 bis 6 Kohlenstoffatomen, eine Alkoxygruppe mit 1 bis 6 Kohlenstoffatomen oder Trifluormethyl darstellen,
- R Wasserstoff, ein Kation, eine Alkylgruppe mit 1 bis 6 Kohlenstoffatomen darstellt,
- n für 0 oder 1 steht,

und ihrerpharmazeutisch akzeptablen Salze zur Herstellung von pharmazeutischen Zusammensetzungen, die insbesondere zur Behandlung von Krampfzuständen und Allergien nützlich sind.